# FASCICULE DE BREVET EUROPEEN

(11) **EP 1 275 345 B1**
(45) Date de publication et mention de la délivrance du brevet: **14.06.2006**
(21) Numéro de dépôt: 02356129.3
(22) Date de dépôt: 05.07.2002
(51) Int. Cl.: A61B 17/17

(54) **Ancillaire de pose d'un composant cubital et/ou d'un composant radial de prothèse de coude**
Hilfsvorrichtung zur Positionierung eines Elle- und/oder Speicheteils einer Ellenbogenprothese
Aid for positioning an ulnar and/or radial component of an elbow prosthesis

(30) Priorité: 09.07.2001 FR 0109100
(43) Date de publication de la demande: 15.01.2003
(73) Titulaire: TORNIER, 38330 Saint Ismier (FR)
(72) Inventeur: Tornier, Alain, 38330 Saint Ismier (FR)
(74) Mandataire: Myon, Gérard Jean-Pierre

(56) Documents cités:
- EP-A- 0 132 284
- EP-A- 1 051 954
- US-A- 4 718 414
- US-A- 5 779 709

## Description

L'invention a trait à un ancillaire de pose d'un composant cubital et/ou d'un composant radial d'une prothèse totale ou partielle de coude

Une prothèse de coude, telle que connue de EP-A-1 051 954, comprend un composant cubital et un composant radial qui doivent reproduire les surfaces articulaires cubitale et radiale. Le positionnement de ces surfaces par rapport à l'articulation, tout particulièrement par rapport à la trochlée humérale, est essentiel pour un bon fonctionnement du coude appareillé.

Jusqu'à présent, les chirurgiens implantent les composants cubitaux et radiaux de prothèse de coude après avoir luxé l'articulation et en tentant de reproduire la configuration anatomique des surfaces articulaires. Il arrive que cette reproduction ne soit pas parfaite, d'où une mauvaise coopération des surfaces articulaires cubitale et/ou radiale avec la trochlée humérale. Ceci conduit à une usure prématurée de ces surfaces et à une gêne pour les patients.

Il est par ailleurs connu de US-A-4,718,414 (qui sert comme base pour le préambule de la revendication 1) ou de EP-A-0132 284 d'utiliser un guide de découpe en vue de poser une prothèse d'épaule. Un tel guide induit un positionnement parfois imprécis des coupes, ce qui implique également une reproduction imparfaite de la configuration anatomique des surfaces articulaires.

C'est à ces inconvénients qu'entend plus particulièrement remédier l'invention en proposant un ancillaire de pose qui permet un positionnement précis du composant cubital et/ou du composant radial par rapport à la trochlée humérale, ce qui améliore sensiblement les conditions de fonctionnement du coude appareillé.

Dans cet esprit, l'invention concerne un ancillaire de pose d'un composant cubital et/ou d'un composant radial d'une prothèse totale ou partielle de coude qui comprend un guide de découpe du cubitus et/ou du radius, ce guide étant destiné à être positionné par rapport à une trochlée humérale fantôme appartenant également audit ancillaire et représentative de la trochlée humérale anatomique, lorsque la trochlée fantôme est en place par rapport aux surfaces articulaires cubitale et/ou radiale correspondantes. Ce guide est également apte à être positionné par rapport au cubitus et/ou radius lorsque la trochlée fantôme est en place par rapport à sa ou leurs surface(s) articulaire(s).

Grâce à l'invention, le guide permet au chirurgien de repérer les zones de resection du ou des os en tenant compte de l'encombrement réel de la trochlée. En d'autres termes, le guide de découpe fonctionne comme un palpeur anatomique tri-dimensionnel de la surface de la trochlée, ce qui permet un positionnement efficace des zones de coupe par rapport à cette trochlée.

Selon des aspects avantageux mais non obligatoires de l'invention, l'ancillaire incorpore une ou plusieurs des caractéristiques suivantes :
- Le guide comprend un support sur lequel peut être montée la trochlée fantôme et sur lequel sont montées des vis aptes à venir en appui contre le cubitus et/ou le radius pour l'immobilisation du guide par rapport à ces os.
- Le support précité comprend une patte dont l'extrémité forme un organe de guidage d'un outil de découpe de l'extrémité supérieure du radius. Cet organe de guidage peut comprendre une fente de coulissement d'une lame de resection de l'extrémité supérieure du radius.
- Le support précité forme un organe cylindrique de guidage d'une scie cloche pour la préparation de l'extrémité supérieure du cubitus.
- Le support précité comprend une patte qui soutient un arbre sur lequel peut être montée la trochlée fantôme. Dans ce cas, l'organe cylindrique de guidage de la scie cloche et l'arbre sur lequel peut être montée la trochlée fantôme ont avantageusement des axes de symétrie confondus, l'arbre précité étant creux et formant un volume de coulissement et de guidage d'un axe central de la scie cloche.
- Le support précité se prolonge par une tige apte à être positionnée, sur la plus grande partie de sa longueur, parallèlement à l'avant-bras du patient. Cette tige permet au chirurgien de pré-positionner rapidement le support avant un réglage fin obtenu grâce aux vis précitées.
- Le support est formé par assemblage de plusieurs pièces sélectionnées en fonction de la taille de la trochlée fantôme. En d'autres termes, la géométrie du support dépend de la taille de la trochlée fantôme, c'est-à-dire en réalité de la taille de la trochlée anatomique dont la trochlée fantôme est une représentation tridimensionnelle. Cette adaptation à la taille de la trochlée anatomique permet une adaptation correspondante des zones de découpe cubitale et radiale.
- Un outil de guidage est prévu pour le perçage du cubitus, cet outil comprenant un gabarit apte à venir en appui contre la surface découpée de l'olécrane, ce gabarit étant pourvu d'un perçage de guidage d'un foret ou analogue, alors que ce gabarit est solidaire d'une poignée de réglage de sa position angulaire par rapport à un axe central de la surface découpée.

L'invention permet la pose d'un composant cubital ou d'un composant radial de prothèse de coude selon un procédé qui comprend des étapes consistant à positionner un guide de découpe par rapport à une trochlée humérale fantôme représentative de la trochlée humérale anatomique, lorsque la trochlée fantôme est en place par rapport aux surfaces articulaires cubitale et/ou radiale correspondantes, à immobiliser ce guide par rapport au cubitus et/ou radius et à découper les extrémités supérieures cubitale et/ou radiale, en fonction de profils définis par ce guide.

On peut en outre prévoir que l'on découpe le cubitus en formant une surface inscrite dans un cylindre et centrée sur un axe géométrique de la surface externe de la trochlée fantôme. Dans ce cas, la trochlée fantôme est avantageusement maintenue en position par rapport au cubitus lors de sa découpe, la découpe ayant lieu, pour partie au moins, autour de cette trochlée fantôme.

En outre, la découpe du cubitus peut être effectuée au moyen d'une scie cloche guidée dans un organe pourvu d'un anneau de guidage co-axial avec la trochlée fantôme.

Enfin, selon un aspect avantageux de ce procédé, l'immobilisation du guide par rapport au cubitus a lieu grâce à trois vis traversant un support du guide et venant respectivement en appui sur l'olécrane, sur le triangle plan postérieur et sur le plan latéral du cubitus.

L'invention sera mieux comprise et d'autres avantages de celle-ci apparaîtront plus clairement à la lumière de la description qui va suivre d'un mode de réalisation d'un ancillaire conforme à son principe et de son procédé d'utilisation, donnée uniquement à titre d'exemple et faite en référence aux dessins annexés dans lesquels :
- La figure 1 est une vue en perspective d'un ancillaire conforme à l'invention, lors d'une première étape d'utilisation, le cubitus et le radius étant représentés en traits fins pour la clarté du dessin ;
- La figure 2 est une vue en perspective de l'ancillaire de la figure 1, lors d'une seconde étape d'utilisation ;
- La figure 3 est une vue analogue à la figure 1 lors d'une troisième étape d'utilisation ;
- La figure 4 est une coupe partielle de principe selon la ligne IV-IV à la figure 3 ;
- La figure 5 est une vue en perspective des extrémités supérieures du radius et du cubitus, lors d'une quatrième étape d'utilisation et

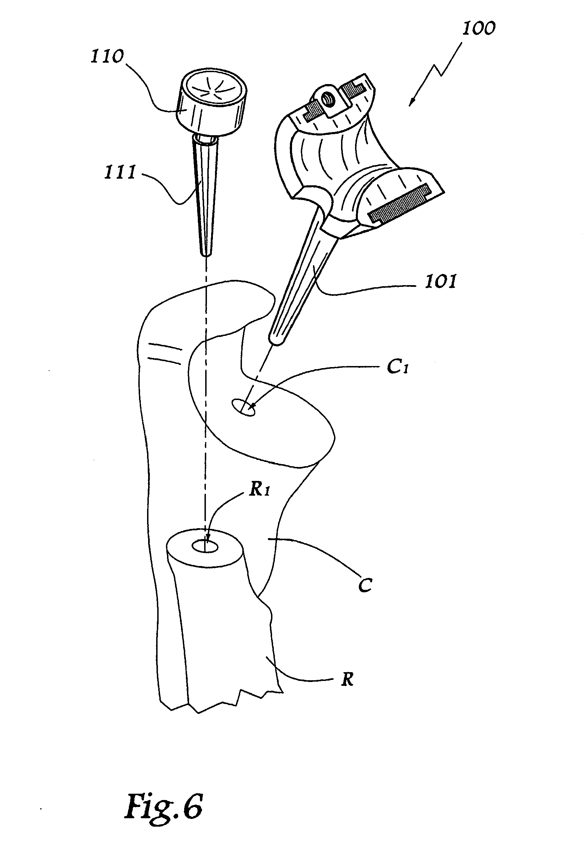

- La figure 6 est une vue analogue à la figure 5 lors de la mise en place de composants prothétiques sur le cubitus et sur le radius.

L'ancillaire 10 conforme à l'invention est destiné à être immobilisé par rapport au cubitus C et au radius R d'une articulation de coude à équiper d'un composant prothétique cubital et/ou d'un composant prothétique radial.

Cet ancillaire comprend plusieurs fantômes d'ensembles articulaires huméraux 20₁, 20₂ et 20₃ que l'on nomme « trochlées humérales fantômes » pour simplifier et que le chirurgien peut comparer successivement à la trochlée anatomique de l'humérus correspondant ou installer dans la cavité sygmoïde du cubitus. Bien entendu, le nombre de trochlées fantômes n'est pas limité à trois et dépend de la précision recherchée lors de cette comparaison.

Les trochlées 20₁, 20₂, 20₃, sont à symétrie de révolution et chacune pourvue d'un perçage central 21 permettant leur montage sur un support 31 formant l'armature d'un guide de découpe 30 appartenant à l'ancillaire 10.

Le support 31 est formé par assemblage mécanique de plusieurs pièces métalliques. Plus précisément, le support 31 comprend un étrier 32 en forme de U sur lequel est monté un arbre creux 33 qui peut être inséré dans le perçage 21 de la trochlée fantôme sélectionnée.

Dans ce qui suit, on suppose que la trochlée 20₂ a été sélectionnée par comparaison avec la trochlée anatomique.

Cette trochlée est alors mise en appui contre la surface interne S_{C} de l'olécrane et la surface articulaire S_{R} de la tête radiale, dans une position proche de celle de la trochlée anatomique avant luxation du coude.

L'arbre 33 est monté sur une première branche 34 de l'étrier 32. La seconde branche 35 de l'étrier 32 se termine par un anneau 36 dont on note l₃₆ la largeur. L'anneau 36 et l'arbre creux 33 sont co-axiaux et centrés sur un axe X-X' qui est également l'axe de symétrie de la trochlée fantôme 20₂ montée sur le support 31.

Le support 31 comprend également une patte 37 immobilisée sur le support 31 et qui comprend deux branches 37a et 37b globalement perpendiculaires l'une par rapport à l'autre, la branche 37b comportant, à son extrémité libre, un guide 37c définissant une fente 37d globalement parallèle à l'axe X-X'.

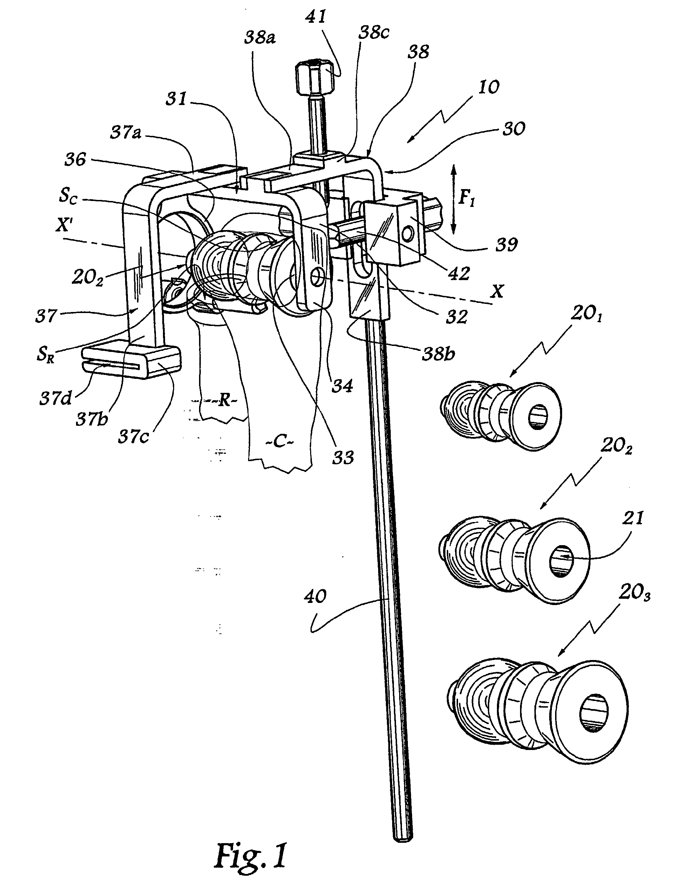

L'étrier 32 supporte également une seconde patte 38 fixée sur l'étrier 31 et qui comprend deux branches 38a et 38b globalement perpendiculaires, l'une par rapport à l'autre. La branche 38b est équipée d'un chariot 39 solidaire d'une tige 40, le chariot 39 pouvant coulisser par rapport à la branche 38b comme représentée par la double flèche F₁ à la figure 1.

La branche 38a est pourvue d'un bossage 38c dans lequel est ménagé un taraudage de réception d'une vis 41 destinée à venir en appui contre la partie proximale de l'olécrane.

Le chariot 39 est, quant à lui, équipé d'une vis 42 destinée à venir en appui contre le triangle plan postérieur du cubitus.

Enfin, l'anneau 36 est pourvu d'une extension 36a dans laquelle est vissée une vis 43 destinée à venir en appui contre le plan latéral du cubitus.

Le guide de découpe 30 est mis en place par rapport au cubitus C et au radius R en disposant la tige 40 globalement le long de l'avant-bras du patient puis en vissant plus ou moins les vis 41 à 43, de telle sorte que la trochlée fantôme 20₂ soit positionnée par rapport aux surfaces articulaires cubitale S_{C} et radiale S_{R} sensiblement de la même façon de la trochlée anatomique lorsque le coude n'est pas luxé. Lorsque cette position est atteinte, les vis 41 à 43 sont serrées, de telle sorte que le guide 30 est immobilisé par rapport au cubitus, et donc par rapport au radius.

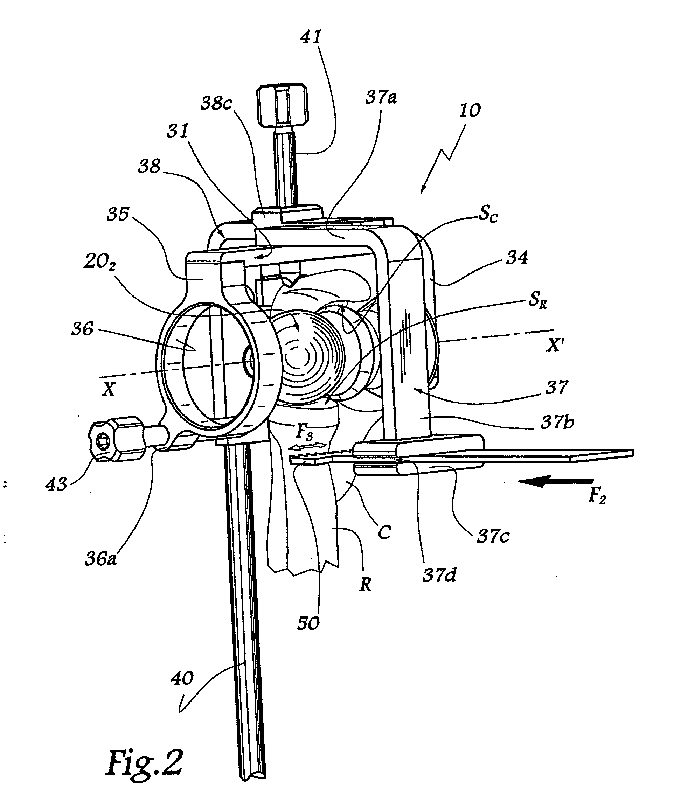

Dans cette position et comme représenté à la figure 2, il est possible d'introduire une lame 50 dans la fente 37d de façon à couper l'extrémité supérieure du radius R. La position de la ligne de coupe du radius R est déterminée en tenant compte de la hauteur du bouton radial qui sera mis en place ultérieurement sur le radius. Ceci permet de garantir que la surface articulaire de ce bouton radial sera correctement positionnée par rapport à la trochlée avec laquelle elle devra coopérer.

A la figure 2, les flèches F₂ et F₃ représentent les directions d'introduction et de mouvement alternatif de la lame 50 en vue de la découpe du radius R.

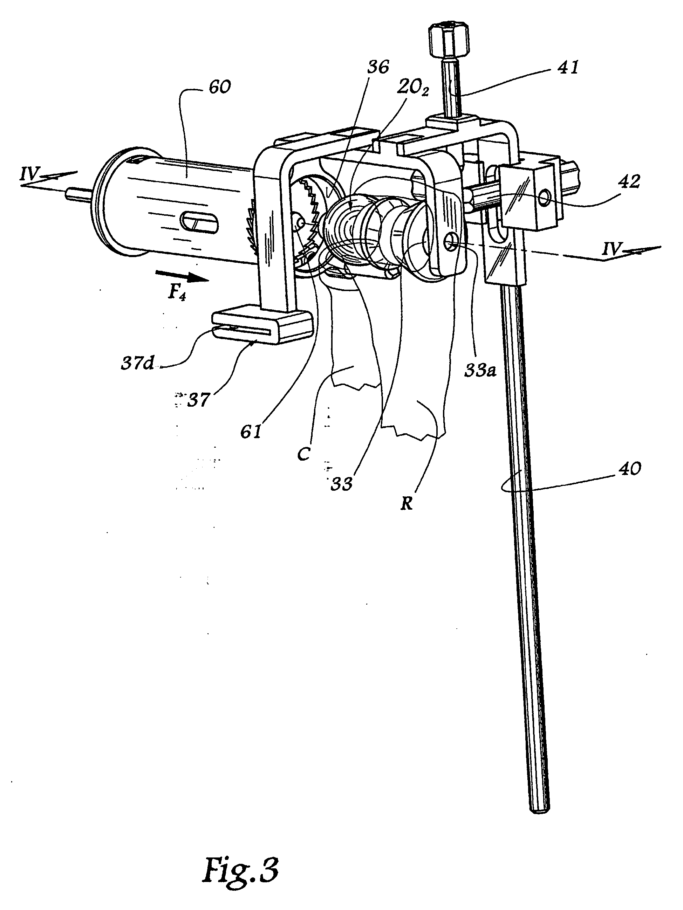

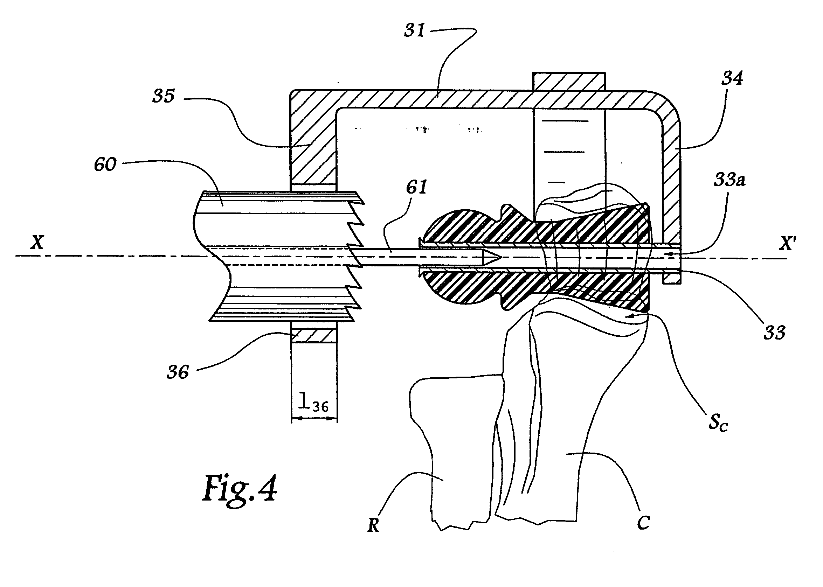

Comme il ressort plus particulièrement des figures 3 et 4, il est alors possible d'introduire une scie cloche 60 dans l'anneau 36, parallèlement à l'axe X-X' et comme représenté par la flèche F₄. La scie cloche 60 est équipée d'un doigt de guidage central 61 aligné sur l'axe X-X' et dont le diamètre lui permet de pénétrer dans le volume intérieur 33a de l'arbre 33, ce qui permet de guider efficacement la scie cloche 60 en coopération avec la surface de portée intérieure de l'anneau 36 qui s'étend sur sa largeur l₃₆.

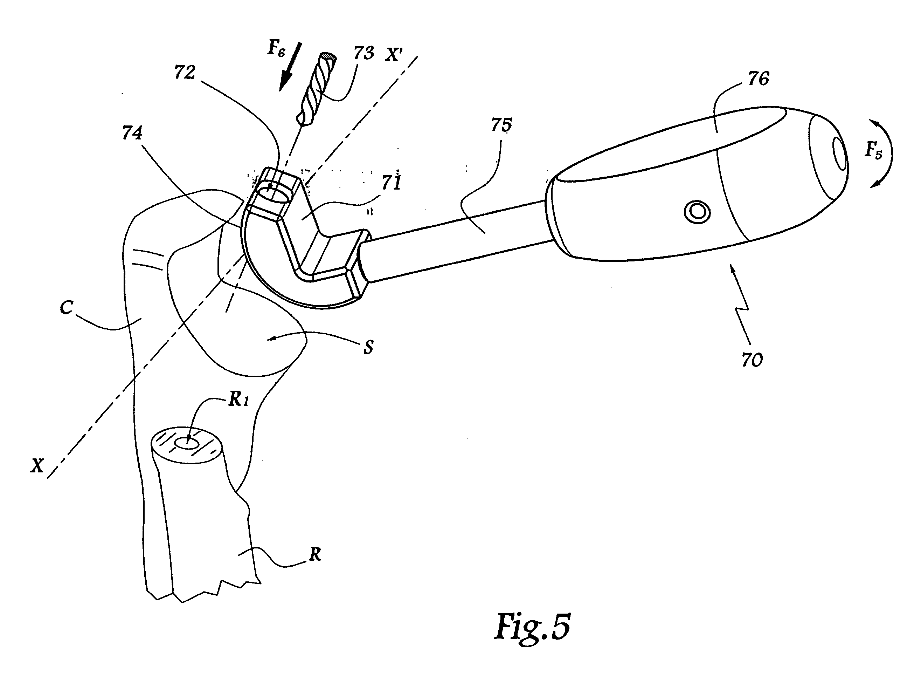

La scie cloche permet de découper l'olécrane du cubitus C autour de la trochlée fantôme en créant une portion de surface cylindrique à base circulaire S, plus particulièrement visible à la figure 5.

Lorsque la découpe du radius et du cubitus a été effectuée, on retire le guide de découpe 30 en desserrant les vis 41 à 43 et l'on peut préparer les os pour la réception des composants prothétiques.

Un perçage R₁ du radius R est effectué sensiblement selon l'axe du col du radius, alors qu'un outil de guidage 70 est utilisé pour déterminer la direction d'un perçage C₁ à effectuer dans le cubitus pour recevoir la queue d'ancrage 101 d'un composant prothétique cubital 100. L'outil 70 comprend un gabarit 71 pourvu d'un perçage 72 de réception et de guidage d'un foret 73, d'un poinçon ou d'un outil de découpe adapté. La surface externe 74 du gabarit 71 est un tronçon de surface cylindrique de même rayon que la surface S et le gabarit 71 peut pivoter autour de l'axe X-X' qui est l'axe de symétrie de la surface S.

Pour commander le positionnement angulaire du gabarit 71 par rapport à l'axe X-X', c'est-à-dire la direction du perçage 72 par rapport au cubitus C, le gabarit 71 est relié par un manche 75 à une poignée 76 destinée à être prise en main par le chirurgien ou l'un de ses assistants et permettant d'exercer un effort de basculement F₅ du gabarit 71 autour de l'axe X-X'.

On comprend que, lorsque le gabarit 71 est en contact avec la surface S, il est possible de créer le perçage C₁ en introduisant le foret 73 dans l'os à travers le perçage 72, comme représenté schématiquement par la flèche F₆.

Lorsque les perçages C₁ et R₁ ont été réalisés, le composant cubital 100 peut être mis en place en introduisant sa queue d'ancrage 101 dans le perçage C₁, alors qu'un bouton radial 110 peut être mis en place sur le radius R en introduisant sa queue d'ancrage 111 dans le perçage R₁.

Grâce à l'invention, il est possible de positionner correctement les composants 100 et 110 en tenant compte de l'anatomie combinée du cubitus, du radius et de l'humérus car, dans la configuration de la figure 1 qui détermine le positionnement du guide 30 et des zones de coupe, la trochlée fantôme est en appui sur les surfaces articulaires anatomiques du cubitus et du radius. Il en résulte un positionnement précis de ces composants, avec une bonne balance ligamentaire de l'articulation.

L'invention a été décrite lors de la mise en place d'un composant cubital et d'un bouton radial mais elle peut bien entendu être utilisée pour la mise en place d'un seul de ces composants.

L'invention a été décrite pour une utilisation par une voie d'abord latérale, en particulier pour un accès de la scie cloche 60 par le côté latéral de l'articulation. Bien entendu, une voie d'abord médiale peut être utilisée, avec un ancillaire conçu symétriquement par rapport à celui représenté sur les figures.

## Revendications

1. Ancillaire de pose d'un composant cubital et/ou d'un composant radial d'une prothèse totale ou partielle de coude qui comprend un guide de découpe (30) du cubitus (C) et/ou du radius (R), **caractérisé en ce que** ledit guide est destiné à être positionné par rapport :
- à une trochlée humérale fantôme (20₂) appartenant également audit ancillaire et représentative de la trochlée humérale anatomique, lorsque ladite trochlée fantôme est en place par rapport aux surfaces articulaires cubitale (S_{c}) et/ou radiale (S_{R}) correspondantes, et
- au cubitus (C) et/ou au radius (R) lorsque ladite trochlée fantôme est en place par rapport à sa ou leurs surface(s) articulaire(s).

2. Ancillaire selon la revendication 1, **caractérisé en ce que** ledit guide (30) comprend un support (31) sur lequel peut être montée ladite trochlée fantôme (20₂) et sur lequel sont montées des vis (41-43) aptes à venir en appui contre le cubitus (C) et/ou le radius (R) pour l'immobilisation dudit guide par rapport auxdits os.

3. Ancillaire selon la revendication 2, **caractérisé en ce que** ledit support (31) comprend une patte (37) dont l'extrémité forme un organe de guidage (37c) d'un outil de découpe (50) de l'extrémité supérieure du radius (R).

4. Ancillaire selon la revendication 3, **caractérisé en ce que** ledit organe de guidage (37c) comprend une fente (37d) de coulissement (F₂, F₃) d'une lame (50) de resection de l'extrémité supérieure du radius (R).

5. Ancillaire selon l'une des revendications 2 à 4, **caractérisé en ce que** ledit support (31) forme un organe cylindrique (36) de guidage d'une scie cloche (60) pour la préparation de l'extrémité supérieure du cubitus (C).

6. Ancillaire selon l'une des revendications 2 à 5, **caractérisé en ce que** ledit support (31) comprend une patte (34) qui soutient un arbre (33) sur lequel peut être montée ladite trochlée fantôme (20₂).

7. Ancillaire selon les revendications 4 et 6,
**caractérisé en ce que** ledit organe cylindrique de guidage (36) et ledit arbre (33) ont des axes de symétrie (X-X') confondus, ledit arbre étant creux et formant un volume (33a) de coulissement et de guidage d'un axe central (61) de ladite scie cloche.

8. Ancillaire selon l'une des revendications 2 à 7, **caractérisé en ce que** ledit support (31) se prolonge par une tige (40) apte à être positionnée, sur la plus grande partie de sa longueur, parallèlement à l'avant bras (C, R) du patient.

9. Ancillaire selon l'une des revendications 2 à 8, **caractérisé en ce que** ledit support (31) est formé par assemblage de plusieurs pièces (32, 37, 38) sélectionnées en fonction de la taille de ladite trochlée fantôme (20₂).

10. Ancillaire selon l'une des revendications précédentes, **caractérisé en ce qu'**il comprend un outil de guidage (70) pour le perçage du cubitus (C), ledit outil comprenant un gabarit (71) apte à venir en appui contre la surface découpée (S) de l'olécrane, ledit gabarit étant pourvu d'un perçage (72) de guidage d'un foret (73) ou analogue, alors que ledit gabarit est solidaire d'une poignée (76) de réglage de la position angulaire dudit gabarit par rapport à un axe central (X-X') de ladite surface découpée.

## Claims

1. Aid for positioning an ulnar and/or radial component of a full or partial elbow prosthesis, incorporating a guide (30) for excising the ulna (C) and/or the radius (R), **characterised in that** said guide is designed to be placed in position with respect:
- to a dummy trochlea of the humerus (20₂) which likewise forms part of said aid and is representative of the anatomical trochlea of the humerus, when said dummy trochlea is in place relative to the corresponding articular surfaces of the ulnus (S_{c}) and/or radius (S_{R}), and
- to the ulna (C) and/or the radius (R) when said dummy trochlea is in place with respect to the articular surface(s) thereof.

2. Aid according to claim 1, **characterised in that** said guide (30) incorporates a support (31) upon which said dummy trochlea (20₂) may be mounted and upon which screws (41-43) are mounted which are adapted to be brought up against the ulna (C) and/or the radius (R) for the immobilisation of said guide with respect to said bones.

3. Aid according to claim 2, **characterised in that** said support (31) incorporates a bracket (37) the end of which forms a guide member (37c) of a tool (50) for excising the upper extremity of the radius (R).

4. Aid according to claim 3, **characterised in that** said guide member (37c) incorporates a slot (37d) in which runs (F₂, F₃) a blade (50) for resecting the upper extremity of the radius (R).

5. Aid according to any one of claims 2 to 4, **characterised in that** said support (31) forms a cylindrical member (36) for guiding a bell saw (60) for the preparation of the upper extremity of the ulna (C).

6. Aid according to any one of claims 2 to 5, **characterised in that** said support (31) incorporates a bracket (34) which supports a shaft (33) upon which said dummy trochlea (20₂) can be mounted.

7. Aid according to claims 4 and 6, **characterised in that** said cylindrical guide member (36) and said shaft (33) have merged axes of symmetry (X-X'), said shaft being hollow and forming a space (33a) in which a central axle (61) of said bell saw runs and is guided.

8. Aid according to any one of claims 2 to 7, **characterised in that** said support (31) is extended by a rod (40) adapted to be positioned, over the great majority of its length, parallel with the patient's forearm (C, R).

9. Aid according to any one of claims 2 to 8, **characterised in that** said support (31) is formed by assembling together a plurality of parts (32, 37, 38) selected according to the size of said dummy trochlea (20₂).

10. Aid according to any one of the preceding claims, **characterised in that** it incorporates a guiding tool (70) for drilling the ulna (C), said tool incorporating a template (71) adapted to be brought up against the excised surface (S) of the olecranon, said template being provided with a drilled hole (72) for guiding a drill bit (73) or the like, whereas said template is rigidly joined to a handle (76) for adjusting the angular position of said template with respect to a central axis (X-X') of said excised surface.

## Patentansprüche

1. Hilfsvorrichtung zum Anbringen eines Cubital- und/oder eines Radialbauteils einer totalen oder teilweisen Ellbogenprothese, die eine Führung zum Ausschneiden (30) des Cubitus (C) und/oder des Radius (R) aufweist, **dadurch gekennzeichnet, dass** die Führerung dazu bestimmt ist, positioniert zu werden in Bezug auf:
- eine Phantom-Humeralisrolle (20₂), die ebenfalls zu der Hilfsvorrichtung gehört und die anatomische Humeralisrolle darstellt, wenn die Phantom-Humeralisrolle in Bezug auf die entsprechende Cubitus-Gelenkfläche (S_{C}) und/oder die Radius-Gelenkfläche (S_{R}) angebracht ist,
- den Cubitus (C) und/oder den Radius (R), wenn die Phantom-Humeralisrolle in Bezug auf ihre Gelenkfläche oder Gelenkflächen angebracht ist.

2. Hilfsvorrichtung nach Anspruch 1, **dadurch gekennzeichnet, dass** die Führung (30) einen Träger (31) umfasst, auf den die Phantom-Humeralisrolle (20₂) montiert werden kann und auf den Schrauben (41-43) montiert sind, die gegen den Cubitus (C) und/oder den Radius (R) zum Stillstellen der Führung in Bezug auf die Knochen zum Aufliegen kommen können.

3. Hilfsvorrichtung nach Anspruch 2, **dadurch gekennzeichnet, dass** der Träger (31) ein Bügel (37) aufweist, deren Ende ein Führungsorgan (37c) eines Ausschneidewerkzeugs (50) des oberen Endes des Radius (R) bildet.

4. Hilfsvorrichtung nach Anspruch 3, **dadurch gekennzeichnet, dass** das Führungsorgan (37c) einen Schlitz (37d) zum Gleiten (F₂, F₃) einer Klinge (50) zur Resektion des oberen Endes des Radius (R) aufweist.

5. Hilfsvorrichtung nach einem der Ansprüche 2 bis 4, **dadurch gekennzeichnet, dass** der Träger (31) ein zylindrisches Teil (36) zum Führen einer Lochsäge (60) zum Vorbereiten des oberen Endes des Cubitus (C) bildet.

6. Hilfsvorrichtung nach einem der Ansprüche 2 bis 5, **dadurch gekennzeichnet, dass** der Träger (31) ein Bügel (34) aufweist, die eine Welle (33) stützt, auf die die Phantom-Humeralisrolle (20₂) montiert werden kann.

7. Hilfsvorrichtung nach den Ansprüchen 4 und 6, **dadurch gekennzeichnet, dass** das zylindrische Führungsorgan (36) und die Welle (33) zusammenfallende Symmetrieachsen (X-X') haben, wobei die Welle hohl ist und ein Volumen (33a) zum Gleiten und Führen einer zentralen Achse (61) der Lochsäge bildet.

8. Hilfsvorrichtung nach einem der Ansprüche 2 bis 7, **dadurch gekennzeichnet, dass** sich der Träger (31) in einen Schaft (40) verlängert, der auf dem größten Teil seiner Länge parallel zu dem Vorderarm (C, R) des Patienten positioniert werden kann.

9. Hilfsvorrichtung nach einem der Ansprüche 2 bis 8, **dadurch gekennzeichnet, dass** der Träger (31) aus dem Zusammenbauen mehrerer Teile (32, 37, 38) gebildet ist, die in Abhängigkeit von der Größe der Phantom-Humeralisrolle (20₂) ausgewählt sind.

10. Hilfsvorrichtung nach einem der vorhergehenden Ansprüche, **dadurch gekennzeichnet, dass** sie ein Führungswerkzeug (70) zum Bohren des Cubitus (C) aufweist, wobei das Werkzeug eine Schablone (71) umfasst, die gegen die ausgeschnittene Fläche (S) des Olekranons zum Aufliegen kommen kann, wobei die Schablone mit einer Bohrung (72) zum Führen eines Bohrers (73) oder dergleichen versehen ist, während die Schablone fest mit einem Griff (76) zum Einstellen der Winkelposition der Schablone in Bezug auf eine zentrale Achse (X-X') der ausgeschnittenen Fläche verbunden ist.
